# EUROPEAN PATENT SPECIFICATION

(11) **EP 3 527 243 B1**
(45) Date of publication and mention of the grant of the patent: **22.12.2021**
(21) Application number: 19157718.8
(22) Date of filing: 18.02.2019
(51) Int. Cl.: A61M 5/20, G09B 23/28

(54) **TRAINER DEVICE FOR AN AUTOINJECTOR**
ÜBUNGSVORRICHTUNG FÜR DEFIBRILLATOREN
DISPOSITIF D'ENTRAINEMENT POUR UN INJECTEUR AUTOMATIQUE

(30) Priority: 19.02.2018 IN 201821006200; 21.08.2018 IN 201821031340
(43) Date of publication of application: 21.08.2019
(73) Proprietor: Wockhardt Limited, Aurangabad 431006 (IN)
(72) Inventor: SHANMUGAM, Loganathan, 560097 Bangalore (IN)
(74) Representative: Nederlandsch Octrooibureau

(56) References cited:
- WO-A1-2017/060017
- WO-A1-2018/007448
- US-A1- 2007 111 175

## Description

### FIELD

The present application is directed to a trainer device for an autoinjector, and particularly, a trainer device wherein the housing is partially received within the cover member.

### BACKGROUND

Autoinjectors have become the main drug delivery devices of choice due to their ability to simplify the number of steps required for injection. However, many patients make mistakes using the devices such as not holding the device correctly or not keeping it in place for long enough. Common errors made by patients include failing to hold the device correctly, not choosing a suitable injection site and not pressing the device hard enough to trigger release of the drug.

To reduce user errors and anxiety, and to build patient confidence, medical device training tools have been developed as trainer devices to support patients in learning how to use their drug delivery devices properly.

Designing and developing an autoinjector trainer can be quite challenging with considerations that do not appear at the surface. The device needs to contain almost all of the elements of the active device, and also include the ability to completely reset the mechanism back to the original state. Not only does the training device need to function within the operational requirements of the active device, but it also needs to perform repeatedly for many cycles depending on the customer requirements.

U.S. Patent Nos. 5,037,306 and 7,682,155 disclose a training device for an autoinjector.

### SUMMARY

The invention is as defined in independent claim 1.

One embodiment discloses a trainer device for an autoinjector comprising: (i) a housing; (ii) a collet holder that includes a track; (iii) a safety cap removably connected to the collet holder; (iv) a cover member; (v) a flex stem having flex arm; and (vi) a cover member locking mechanism having a first locking position and a second locking position, wherein the housing is partially received inside the cover member, and the flex arm of the flex stem traverses the track during actuation of the trainer device upon removal of the safety cap, wherein the cover member locking mechanism is as defined in claim 1.

Some embodiments disclose a trainer device for an autoinjector comprising: a housing; a cover member; and a cover member locking mechanism comprises cam profiles and cam follower nuts, wherein cam profiles are provided on the cam surfaces of housing and cam follower nuts are positioned between the cover member and the housing in both first locking position and second locking position.

Some embodiments disclose a trainer device for an autoinjector comprising: a housing; a cover member; and a cover member locking mechanism includes cam profiles and cam follower nuts, wherein the cam profile has five vertices and comprises a first angled cam track, a vertical cam track and a second angled cam track, wherein (i) the first angled cam track is formed by traversing of the cam profile from the first vertex to second and third vertices towards the proximal direction; (ii) the vertical cam track is formed by traversing of the cam profile from the third vertex to fourth vertex in the distal direction; and (iii) the second angled cam track is formed by traversing the cam profile from the fourth and fifth vertices to first vertex again in the proximal direction.

Some embodiments disclose a trainer device for an autoinjector comprising: a housing; a cover member; and a cover member locking mechanism includes cam profiles and cam follower nuts, wherein the cam profiles comprise two integral snap lock members which include (i) first integral snap lock member formed by groove cut on the surface of first angled cam track, and (ii) second integral snap lock member formed by groove cut on the surface of vertical cam track.

Some embodiments disclose a trainer device for an autoinjector comprising: a housing; a cover member; and a cover member locking mechanism includes cam profiles and cam follower nuts, wherein the cam follower nuts comprise a cam follower protrusion on the distal end portion; a cam follower button on the proximal end portion; and a cam follower body extending between protrusion and button, wherein the protrusion and button are extending opposite to each other.

Some embodiments disclose a trainer device for an autoinjector comprising: (i) a housing; (ii) a collet holder; (iii) a safety cap removably connected to the collet holder; (iv) a flex stem having flex arm; (v) a cover member, and (vi) a cover member locking mechanism having a first locking position and a second locking position, wherein in the first locking position, the cover member is in the retracted position; and in the second locking position, the cover member is in the extended position; said cover member locking mechanism comprising cam profiles and cam follower nuts, wherein the cam profiles are provided on the cam surfaces of housing and cam follower nuts are positioned between the cover member and the housing in both first locking position and second locking position; and (vii) a resetting mechanism comprising locking projections provided on the housing and reset members provided on the cover member, wherein the resetting of cover member results in proximal movement of cover member to the retracted position; said proximal movement is allowed by pressing the reset members that release the locking connection between the reset members and locking projections.

Some embodiments disclose a trainer device for an autoinjector comprising: a housing; a collet holder that includes a track; a safety cap removably connected to the collet holder; a cover member; and a flex stem having flex arm, wherein the collet holder comprises (i) single piece body defining longitudinal central axis having opposed upper and bottom surfaces, (ii) opposed open distal end and a closed proximal end, (iii) a pair of laterally opposed sides, and (iv) an upper core member with a top planar surface perpendicular to longitudinal central axis, wherein the upper surface of the collet holder has a stop wall and a recess, wherein the recess extends longitudinally from the open distal end to the stop wall and defining the track for the flex arm.

Some embodiments disclose a trainer device for an autoinjector comprising: a housing and a cover member, wherein the cover member further comprises a sleeve which is at least partially surrounds the outer surface of the cover member to prevent or inhibit the obstruction of the movement of the cover member when the user accidentally holds the cover member during the actuation of trainer device.

Some embodiments disclose a trainer device comprising a plastic sleeve received on the outer surface of the cover member, the plastic sleeve includes a first end and a second end, wherein the first end and the second end are separated by a coil body extending along a coiled axis.

Some embodiments disclose a trainer device comprising an elastomeric sleeve received on the outer surface of the cover member, the elastomeric sleeve includes a first end and a second end, wherein the first end and the second end are separated by a tubular body.

Some embodiments disclose a trainer device for an autoinjector comprising: a housing and a cover member, wherein the cover member comprises a base cover member and a top cover member, and wherein the top cover member has concentrically and coaxially arranged an inner sleeve and an outer sleeve.

### BRIEF DESCRIPTION OF THE DRAWINGS

FIGURE 1 shows a side cross-sectional view of the trainer device before actuation;
FIGURE 2 shows a side perspective view of the cam profile of the housing;
FIGURE 3 shows a side cross-sectional view of the trainer device during removal of the safety cap;
FIGURE 4 shows a side cross-sectional view of the trainer device during actuation;
FIGURE 5 shows a side cross-sectional view of the trainer device after actuation;
FIGURE 6 shows perspective views of the cam follower nut of the trainer device;
FIGURE 7 shows perspective views of the flex stem and the flex arm of trainer the device;
FIGURE 8 shows a front perspective view of the housing;
FIGURE 9 shows perspective views of the collet holder of the trainer device;
FIGURE 10 shows perspective views and front views of the top cover member and the bottom cover member of the trainer device according to the embodiment of FIGURE 1;
FIGURE 11 shows traversing of the cam follower nut in the cam profile of the trainer device during first locking position, actuation stage, second locking position and after resetting stages of operation;
FIGURE 12 shows a side cross-sectional view of the trainer device before actuation;
FIGURE 13 shows a side cross-sectional view of the trainer device during removal of safety cap;
FIGURE 14 shows a side cross-sectional view of the trainer device during actuation;
FIGURE 15 shows a side cross-sectional view of the trainer device after actuation;
FIGURE 16 shows a perspective view of trainer device wherein the plastic sleeve is partially around the cover member;
FIGURE 17 shows perspective views and front views of the top cover member and the bottom cover member of the trainer device according to the embodiment of FIGURE 12;
FIGURE 18 shows perspective views of the plastic sleeve according to one embodiment;
FIGURES 19a-19e shows assembling procedures of the cover member and the plastic sleeve to the housing of the trainer device according to the embodiment shown in FIGURES 12 to 16;
FIGURE 20 shows perspective views of the elastomeric sleeve according another embodiment;
FIGURE 21 shows a perspective view of trainer device wherein the elastomeric sleeve is partially around the cover member;
FIGURE 22 shows perspective views of the cover member according to yet another embodiment; and
FIGURE 23 shows a perspective view of the trainer device according to the cover member of FIGURE 22

### DETAILED DESCRIPTION

The present application is directed to a trainer device for an autoinjector,
As used herein, the term "distal end" in the appended figures and description is meant to refer to the end of the trainer device carrying the cover member whereas the term "proximal end" is meant to refer to the opposite end carrying the safety cap. The term "second end" in the appended figures and description is meant to refer to the end of the trainer device carrying the cover member whereas the term "first end" is meant to refer to the opposite end carrying the safety cap.

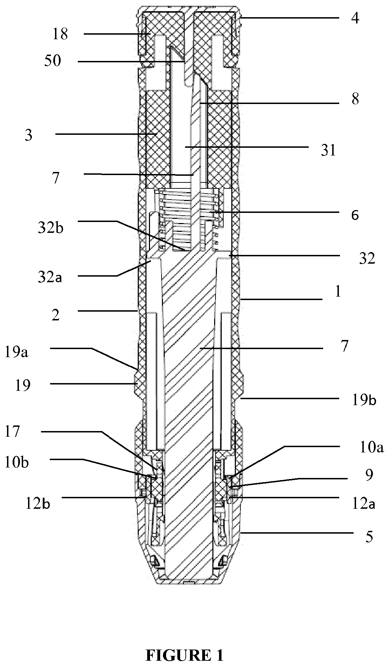

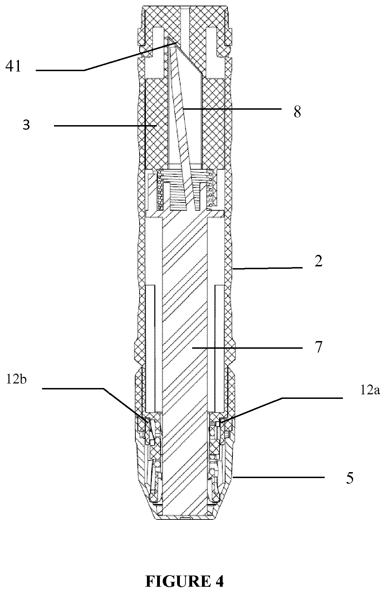

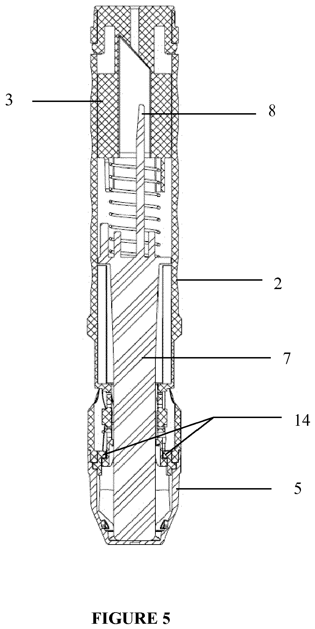

Referring to **FIGURES 1-6****,** a trainer device **1** for an autoinjector, comprising: a housing **2;** a collet holder **3**,a safety cap **4;** a cover member **5;** a flex stem **7;** a flex arm **8**; and a cover member locking mechanism **9,** wherein the housing **2** is partially received inside the cover member **5**. The cover member locking mechanism 9 comprising diametrically opposed cam profiles **10a, 10b** and cam follower nuts **12a, 12b,** wherein the cam follower nuts **12a, 12b** are positioned between the cover member 5 and the housing **2** in both the first locking position **13** and the second locking position **14**. Further the trainer device **1** comprising a spring **6** to bias the flex stem **7;** the spring **6** is positioned between flex stem **7** and collet holder **3**. The position of cover member **5** in the first locking position **13** and the second locking position **14** may be attained by the cover member locking mechanism **9**.

The housing 2 comprising a first sleeve section **16** and a second sleeve section **17**, having opposed proximal and distal end portions, the second sleeve section **17** having reduced diameter than the first sleeve section **16** of the housing **2**. The first sleeve section **16** at the proximal end has an end surface **18** to receive the safety cap **4** and the distal end having a peripheral ridge **19**. The peripheral ridge **19** having front end **19a** and rear end **19b** portions, whereas the first sleeve section **16** is affixed into the front end portion **19a** and the second sleeve section **17** is extending from the rear end portion **19b**. At the rear end **19b,** a pair of diametrically opposed rectangular shaped like projection heads **20a, 20b, 20c, 20d** extending downwardly on the surface of second sleeve section **17**.

The second sleeve section **17** at distal end comprising diametrically opposed trapezium shaped like arms **21a, 21b** has cutting flank surfaces on the outer surface defining the cam surfaces **22a, 22b**. The housing 2 may be cylindrical, oval or elliptical in shape.

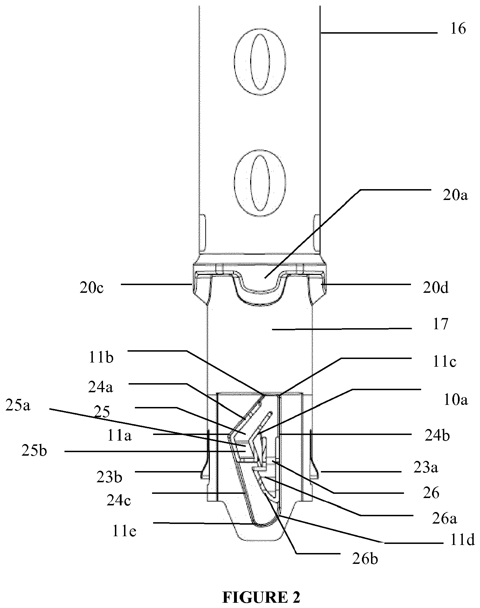

Referring to **FIGURE 2**, the cam profiles **10a, 10b** are provided on the diametrically opposed cam surfaces **22a, 22b** and each of the cam profile **10a** and **10b** is shaped like a pentagon having five vertices **11a, 11b, 11c, 11d,** and **11e**. The cam profiles **10a, 10b** comprising a first angled cam track **24a,** a vertical cam track **24b** and a second angled cam track **24c**. The first angled cam track **24a** is formed by traversing the cam profile from the vertex **11a** to vertices **11b, 11c** towards the proximal direction, a vertical cam track **24b** is formed by traversing the cam profile from the vertex **11c** to vertex **11d** in the distal direction and second angled cam track **24c** is formed by traversing the cam profile from the vertices **11d, 11e** to vertex **11a** again in the proximal direction. The cam profiles **10a, 10b** may be in any polygon-shape for traversing of cam follower nuts **12a, 12b** during the operation of trainer device **1**.

Referring to **FIGURE 2**, wherein each of cam profile **10a** and **10b** comprising two integral snap lock members **25** and **26**, a first integral snap lock member **25** is formed by groove cut on the surface of cam track **24a** and a second integral snap lock member **26** is formed by groove cut on the surface of cam track **24b**. Further, the first snap lock member **25** comprising a protrusion head **25a** towards distal end, which extends radially outward having the sloping surface **25b** on the front projection surface and the second snap lock **26** member comprising a protrusion head **26a** extend radially sideward having sloping surface **26b** on the front projection surface. Both the first and second snap lock members **25**, **26** are separated by a small protrusion raised upwardly. The integral snap lock members **25** and **26** provides the one-way traversing of cam follower nuts **12a** and **12b** in the cam profiles **10a, 10b** during the operation of trainer device **1**, while maintaining the cover member **5** in the first locking position **13** and second locking position **14**.

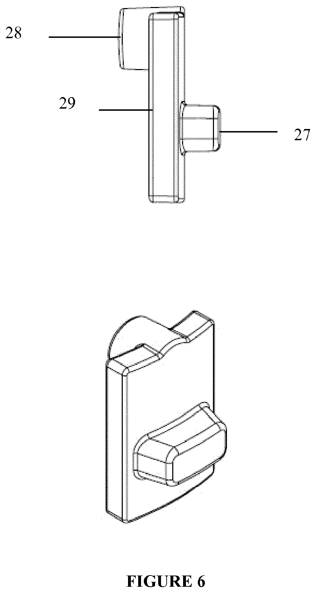

Referring to **FIGURE 6**, the cam follower nuts **12a** and **12b** comprising a cam follower protrusion **27** on the distal end portion and a cam follower button **28** on the proximal end portion, whereas a cam follower body **29** is extending between **27** and **28**. The protrusion **27** and the button **28** are extending opposite to each other from cam follower body **29**.

Referring to **FIGURES 1-6**, the cam profiles **10a**, **10b** on the housing **2** along with the cam follower nuts **12a**, **12b** controls the movement of cover member **5** from the first locking position **13** to the second locking position **14**. In the first locking position **13**, the cover member **5** is in the retracted position and in the second locking position **14**, the cover member **5** is in the extended position. The present disclosure provides single locking mechanism **9** that controls the cover member **5** in both the retracted and extended positions before and after use of trainer device **1**.

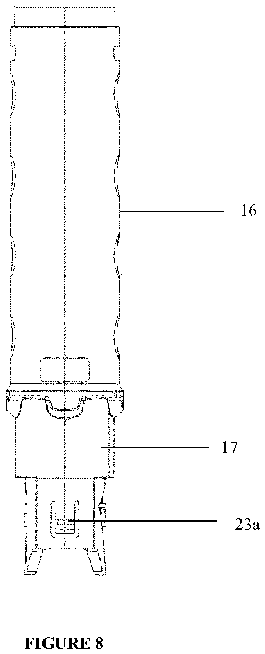

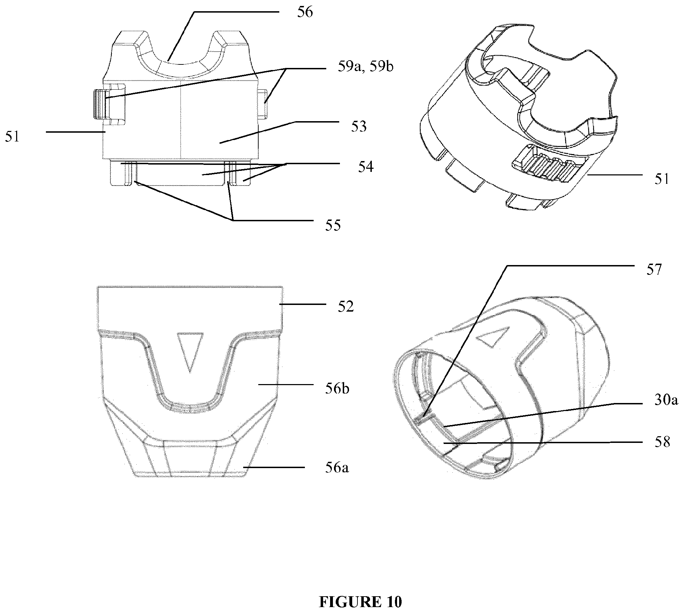

Referring to **FIGURE 2**, and **FIGURE 8**, locking projections **23a**, **23b** formed on the outer surface of second sleeve section **17** by groove cut diametrically opposite to the cam surfaces **22a**, **22b**. The locking projections **23a**, **23b** include a bevelled end surface extending radially outward which is shaped to lock with the reset members **59a**, **59b** (as shown in **FIGURE 10**) of cover member **5** when the cover member **5** is in the extended position.

The cam follower button **28** of the cam follower nuts **12a** and **12b** is engaged with protrusion head **25a** of cam track **24a** in the first locking position **13**. The cam follower button **28** of the cam follower nuts **12a** and **12b** abut with vertex **11d** on the cam track **24b** in the second locking position **14**.

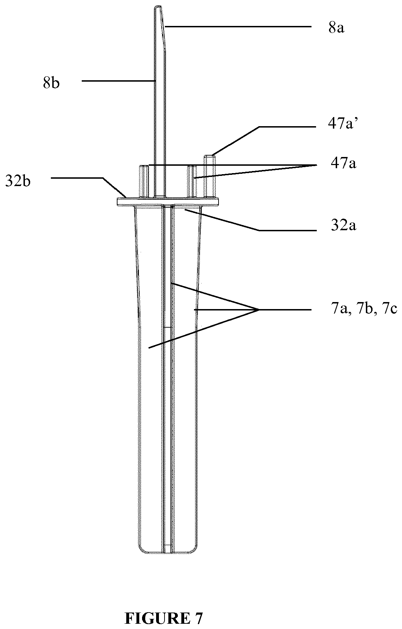

Referring to **FIGURE 1** and **FIGURE** 7, a flex stem 7 comprising a body defining a longitudinal central axis, four longitudinal blades **7a**, **7b**, **7c**, **7d** extending radially outward from the body, and opposed proximal and distal end portions. Further, the flex stem **7** has a base part **32** having a front face **32a** and a rear face **32b**, whereas the four longitudinal blades **7a**, **7b**, **7c**, **7d** affixed to the front face **32a** of the base part **32**.

A flex arm **8** is extending from the rear face **32b** of the flex stem base part **32** towards proximal end. A plurality of ribs **47a** also extend from the rear face **32b** of the flex stem base part **32**. Furthermore, a rib **47a'** extends from the periphery of rear face **32b**.

The flex arm **8** has a blade body having opposed first and second surfaces **8a**, **8b** that extend along a longitudinal axis of blade body with tapered end on the first surface **8a** of the blade body.

Prior to the use of trainer device **1**, the pin **50** of the safety cap **4** abuts with the tapered end of the flex arm **8** and obstructs the traversing of the flex arm **8** on the track **31** of the collet holder **3**.

The flex stem **7** has abutment with the cover member **5** and is held against the spring **6** between the collet holder **3** and cover member **5**, which restricts the movement of cover member **5** prior to the use of trainer device **1**.

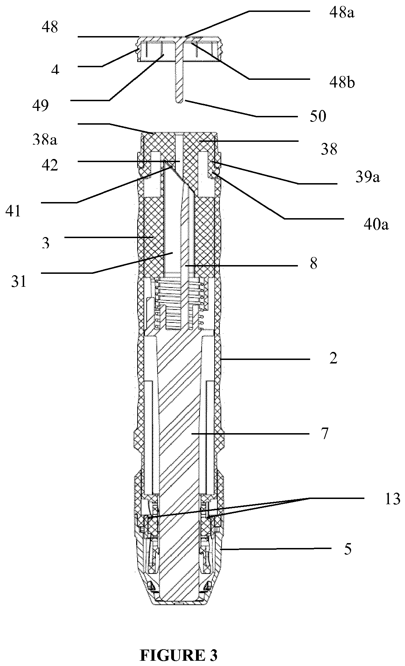

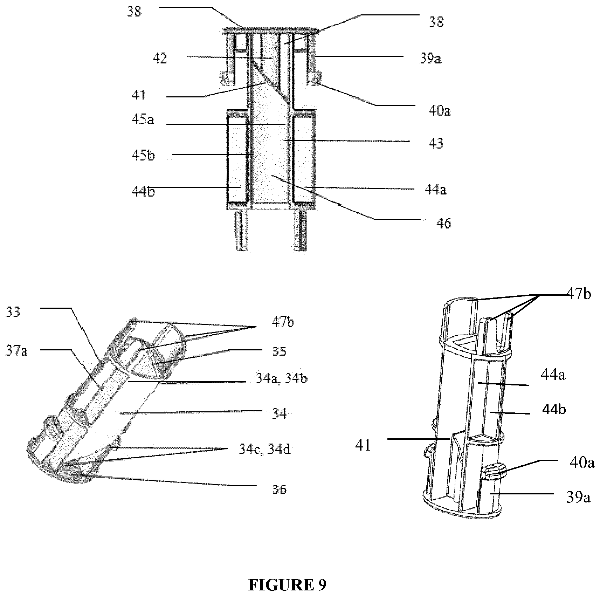

Referring to **FIGURE 3** and **FIGURE 9****,** the collet holder **3** is a single piece body defining longitudinal central axis having, opposed upper and bottom surfaces **33** and **34**, opposed open distal end **35** and closed proximal end **36**, a pair of laterally opposed sides **37a**, **37b**. Further, the collet holder **3** has an upper core member **38** with a top planar surface **38a** perpendicular to longitudinal central axis.

The collet holder **3** further comprises a pair of opposed projecting clips **39a**, **39b** extending downwardly from the upper core member **38** with radially outward protrusion heads **40a** and **40b** at the distal end. The collet holder **3** may be cylindrical, oval or elliptical in shape.

The bottom surface **34** of collet holder **3** having four corners **34a**, **34b**, **34c** and **34d,** and a stop wall **41**. The stop wall **41** extending upwardly from the two corners **34c, 34d** of bottom surface **34** and being deflected outwardly at a region somewhat below the top-platen of collet holder **3** and disposed in common horizontal plane of upper core member **38**. The upper core member **38** has an opening **42** extending in a common horizontal plane at the central axis. Further, the upper core member **38** of the collet holder **3** has an opening **42** to receive the safety cap **4**.

The upper surface **33** of the collet holder **3** has a stop wall **41** towards closed proximal end **36** and a recess **43** extending longitudinally from open distal end **35** to the stop wall **41,** and the stop wall **41** having front contact surface **41a**. The recess **43** with the stop wall **41** defining the track **31** for the flex arm **8**. Furthermore, the recess **43** formed in the upper surface **33** of the collet holder **3** having, a pair of opposed side surfaces **45a**, **45b** and central surface **46** extending parallel to the bottom surface **34** of collet holder **3** and ends in the stop wall **41**. The flex arm **8** at the distal end is in always contact and rests in the front contact surface **41a** of stop wall **41.**

The trainer device **1** of an autoinjector, wherein the track **31** is formed by a recess **43** which extends longitudinally from open distal end **35** to the closed proximal end **36**.

The laterally opposite sides **37a**, **37b** at the distal end **35** of the collet holder **3** are having two inclined surfaces **44a**, **44b** perpendicular to each other.

A pair of diametrically opposed extended ribs **47b** extended from the open distal end **35** of collet holder **3**, wherein one of extended rib **47b** is having curved cross-sectional recess at the middle of the rib body.

Referring to **FIGURE 7** and **FIGURE 9**, the rib **47a'** of flex stem 7 and diametrically opposed extended ribs **47b** of the collet holder **3** define the spring platform **15**, wherein the rib **47a'** fits in the curved cross-sectional recess of one of the extended rib **47b**. The spring platform **15** provides constant holding of spring **6** during the operation of the trainer device **1**.

A safety cap **4** is removably connected to the collet holder **3**. Referring to **FIGURE 1** and **FIGURE 3**, the safety cap **4** comprises a flat top wall **48** having a top planar surface **48a**, a bottom surface **48b**, and a side wall **49.** The side wall **49** extends downwardly from the periphery of flat top wall **48** to the open free end. The side wall has inner surface **49a** and outer surface **49b**. The inner surface **49a** side wall **49** is sized to rest on the end surface of housing **18**. Furthermore, the safety cap **4** has a pin **50** extending downwardly from the bottom surface **48b** of top wall **48** and removably engaged within an opening **42** in the upper core member **38** of the collet holder **3**.

To actuate the trainer device **1**, the user has to remove the safety cap **4** which allows the traversing of the flex arm **8** on the track **31** of the collet holder **3** during the operation.

Referring to **FIGURE 10**, the cover member **5** has two parts, an upper part **51** and a lower part **52**, whereas the upper part **51** and the lower part **52** of cover member **5** are connected via plurality of rib and groove arrangements.

The upper part **51** of cover member **5** comprising, opposed proximal and distal end portions, whereas both the ends having the opening. A side wall **53** extending from a proximal end to a distal end around the periphery of the upper part, a plurality of ribs **54** separated by continuous grooves **55** extending downwardly along an inner circumference of the side wall **53** at the distal end in symmetrical arrangement. Further, a pair of diametrically opposed U-shaped like cross-sectional grooves **56** are provided on the side wall **53** at the proximal end.

The lower part **52** of cover member **5** comprising: opposed open proximal end and closed distal ends. A bottom wall **56a** having a periphery defined the closed distal end and a side wall **56b.** The side wall **56b,** first extending in an annular fashion from the open proximal end followed by tapering frustoconical fashion and nests to periphery of bottom wall **56a.**

A plurality of ribs **57** separated by continuous grooves **58** extending upwardly along the inner circumference of side wall **56b** at the proximal end to receive the corresponding ribs **54** and grooves **55** in the proper orientation of upper part **51** and lower part **52** of cover member **5**. Diametrically opposed ledges **30a, 30b** provided on the inner circumference of side wall **56b** below the grooves **58**. The ledges **30a, 30b** are having two edges. During the operation of trainer device **1**, the protrusion **27** of the cam follower nuts **12a** and **12b** moves rotationally from one edge to another edge of ledge **30a** and **30b.**

The inside diameter of annular side wall **56b** at the proximal end of lower part **52** of cover member **5** is approximately equal to the outer diameter annular side wall **53** at the distal end of upper part **51** of cover member **5**.

Referring to **FIGURE 10**, the reset members **59a**, **59b** are provided on the cover member **5**, more particularly on the upper part **51** of the cover ember **5**. The reset members **59a** and **59b** is an elongated arm having upper and lower surfaces formed by groove cut on the surface of cover member **5**, the end portion of reset members **59a**, **59b** are locked with locking projections **23a**, **23b** of the housing **2** by abutting of lower surface of the reset member with bevelled end surface of locking projection.

For using the device, first the safety cap **4** is removed and placed the cover member 5 on the suitable part of the body, for example the upper leg, and is pressed. The cover member **5** moves backwards towards proximal end which results in a relative backward movement of the flex stem 7. While the flex stem 7 is moved backwards, the flex arm **8** slidably traverses the front contact surface **41a** of stop wall **41** from side surface **45a** to **45b** of recess **43**. After removing the cover member **5** from the body, the cover member **5** is extended forward towards distal end by the action of the spring **6**.

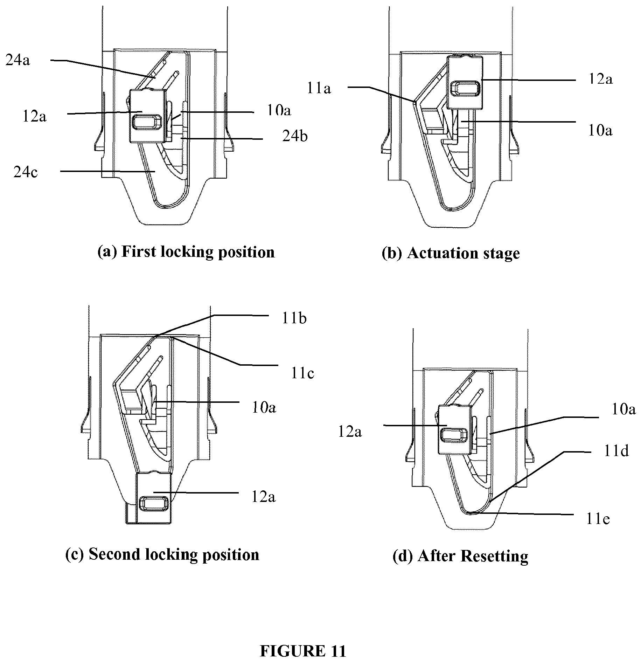

**FIGURE 11** shows the exemplary movement of cam follower nut **12a** on the cam profile **10a** during the various stages of the trainer device **1** for example the first locking position **13**, the actuation stage, the second locking position **14** and the resetting stage. In the first locking position **13,** the cam follower button **28** rests in the vertex **11a** of cam track **24a**. When the cover member **5** is moved backward, the cam follower button **28** traverses in the cam track **24a** from vertex **11a** to **11c** via **11b;** when the cover member **5** extends forward, the cam follower button **28** traverses in the cam track **24b** from vertex **11c** to vertex **11d** and the cover member **5** is in the second locking position **14**. Similarly, the cam follower nut **12b** follows the same movement on the diametrically opposed cam profile **10b.**

After using the trainer device 1, it may be ready for reuse with a resetting mechanism **60**. As shown in **FIGURE 2** and **FIGURE 10****,** the resetting mechanism **60** comprises: locking projections **23a, 23b** provided on the housing **2** and reset members **59a, 59b** provided on the cover member **5**. To reset the device, the user has to first place the safety cap **4** in the end surface **18** of housing **2** and presses the reset members **59a, 59b** on cover member **5**. Once the reset members **59a, 59b** are pressed, the locking connection between the reset members **59a, 59b** and locking projections **23a, 23b** gets released and the bevelled end portion of locking projection easily passes the reset member; then user pushes the cover member **5** back towards the proximal end from the second locking position **14** to first locking position **13** and the trainer device **1** ready for reuse. The present disclosure provides resetting mechanism **60** and is separated from the locking mechanism **9** of cover member **5** and does not disturb the cover member **5** in both retracted and extended positions.

In the trainer device **1** resetting operation, the cover member **5** moves backwards to first locking position **13**, as shown in **FIGURE 11****,** the cam follower button **28** traversing in cam track **24c** from vertex **11d** to **11a** via **11e** by passing of protrusion head **25a** of first snap lock member **25**.

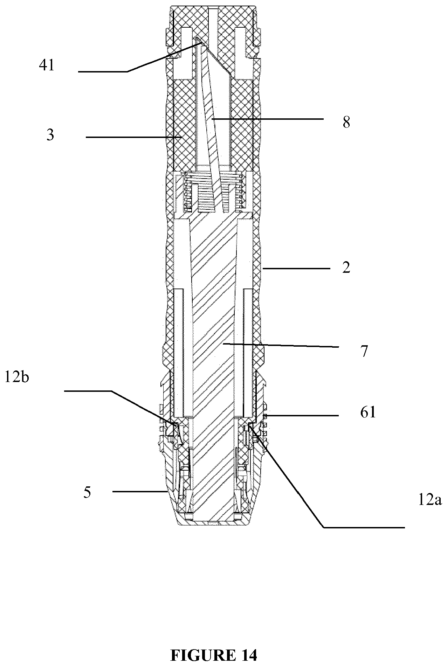

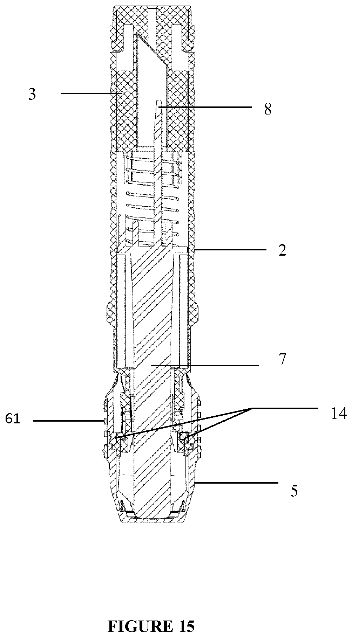

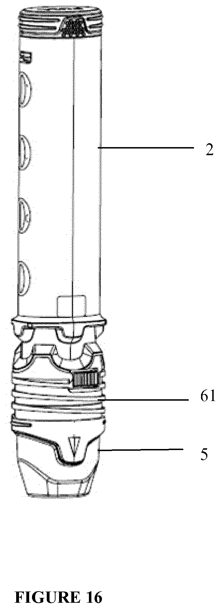

Another embodiment of the invention is described with reference to **FIGURE 2****,** **FIGURE 6****,** **FIGURE 7****,** **FIGURE 8****,** **FIGURE 9****,** **FIGURE 11** and **FIGURES 12****-23,** a trainer device **1** for an autoinjector, comprising: a housing **2**; a collet holder **3**,a safety cap **4**; a cover member **5**; a flex stem 7; a flex arm **8**; a cover member locking mechanism **9**, wherein the housing **2** is partially received inside the cover member **5**; wherein the cover member locking mechanism **9** comprising diametrically opposed cam profiles **10a, 10b** and cam follower nuts **12a, 12b,** wherein the cam follower nuts **12a,12b** are positioned between the cover member **5** and the housing **2** in both first locking position **13** and second locking position **14**. Further the trainer device **1** comprising a spring **6** to bias the flex stem 7; the spring **6** is positioned between flex stem **7** and collet holder **3**. The position of cover member **5** in first locking position **13** and second locking position **14** may be attained by cover member locking mechanism **9.** The cover member **5** further comprises a sleeve, may be observed to be at least partially around the outer surface of the cover member **5**.

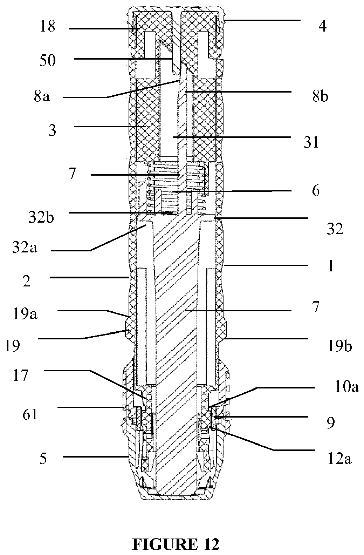

The housing **2** will now be described in greater detail in connection with **FIGURE 2****,** and **FIGURE 12**. The housing **2** comprising a first sleeve section **16** and a second sleeve section **17**, having opposed proximal and distal end portions, the second sleeve section **17** having reduced diameter than the first sleeve section **16** of the housing **2**. The first sleeve section **16** at the proximal end has an end surface **18** to receive the safety cap **4** and the distal end has a peripheral ridge **19**. The peripheral ridge **19** having front end **19a** and rear end **19b** portions, whereas the first sleeve section **16** is affixed into the front end portion **19a** and the second sleeve section **17** extending from the rear end portion **19b**. At the rear end **19b**, a pair of diametrically opposed rectangular shaped like projection heads **20a**, **20b, 20c**, **20d** extending downwardly on the surface of second sleeve section **17**.

The second sleeve section **17** at distal end comprising diametrically opposed trapezium shaped like arms **21a, 21b** which has cutting flank surfaces on the outer surface defining the cam surfaces **22a**, **22b**. The housing **2** may be cylindrical, oval or elliptical in shape.

The cam profiles **10a**, **10b** (as shown in **FIGURE 2** of aforementioned embodiment) are provided on the diametrically opposed cam surfaces **22a**, **22b** and each of the cam profile **10a** and **10b** is shape like a pentagon having five vertices **11a**, **11b**, **11c**, **11d**, and **11e**. The cam profiles **10a**, **10b** comprising a first angled cam track **24a**, a vertical cam track **24b** and a second angled cam track **24c.** The first angled cam track **24a** is formed by traversing the cam profile from the vertex **11a** to vertices **11b**, **11c** towards the proximal direction, a vertical cam track **24b** is formed by traversing the cam profile from the vertex **11c** to vertex **11d** in the distal direction and second angled cam track **24c** is formed by traversing the cam profile from the vertices **11d**, **11e** to vertex **11a** again in the proximal direction. The cam profiles **10a**, **10b** may be in any polygon-shaped for traversing of cam follower nuts **12a**, **12b** during the operation of the trainer device **1**.

Each of cam profile **10a** and **10b** comprising two integral snap lock members **25** and **26** (as shown in **FIGURE 2** of aforementioned embodiment), a first integral snap lock member **25** is formed by groove cut on the surface of cam track **24a** and a second integral snap lock member **26** is formed by groove cut on the surface of cam track **24b**. Further, the first snap lock member **25** comprising a protrusion head **25a** towards distal end extend radially outward having the sloping surface **25b** on the front projection surface and the second snap lock **26** member comprising a protrusion head **26a** extend radially sideward having sloping surface **26b** on the front projection surface. Both the first and second snap lock members **25**, **26** are separated by a small protrusion raised upwardly. The integral snap lock members **25** and **26** provides the one-way traversing of cam follower nuts **12a** and **12b** in the cam profiles **10a**, **10b** during the operation of the trainer device **1**, while maintain the cover member **5** in the first locking position **13** and second locking position **14**.

The cam follower nuts **12a** and **12b** (as shown in **FIGURE 6** of aforementioned embodiment) comprising a cam follower protrusion **27** on the distal end portion and a cam follower button **28** on the proximal end portion, whereas a cam follower body **29** extending between **27** and **28**. The protrusion **27** and the button **28** are extending opposite to each other from the cam follower body **29**.

The cam profiles **10a**, **10b** on the housing **2** along with the cam follower nuts **12a**, **12b** controls the movement of the cover member **5** from the first locking position **13** to the second locking position **14**. In the first locking position **13**, the cover member **5** is in the retracted position and in the second locking position **14**, the cover member **5** is in the extended position. The present disclosure provides single locking mechanism **9** that controls the cover member 5 in both the retracted and extended positions before and after use of trainer device 1.

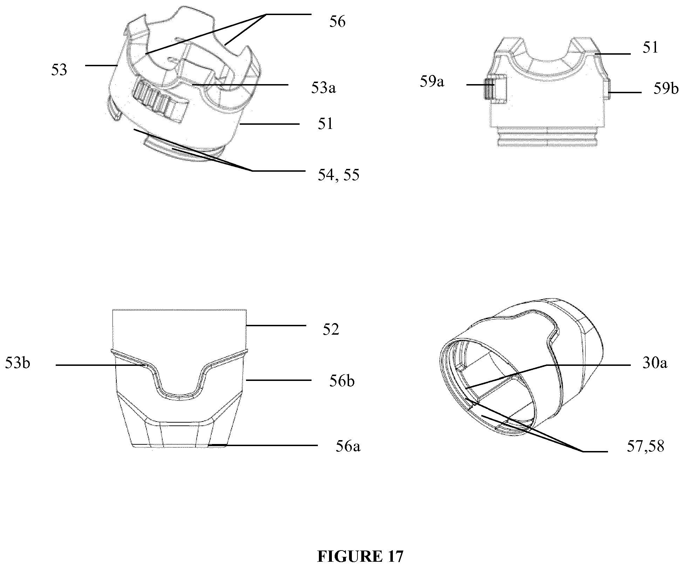

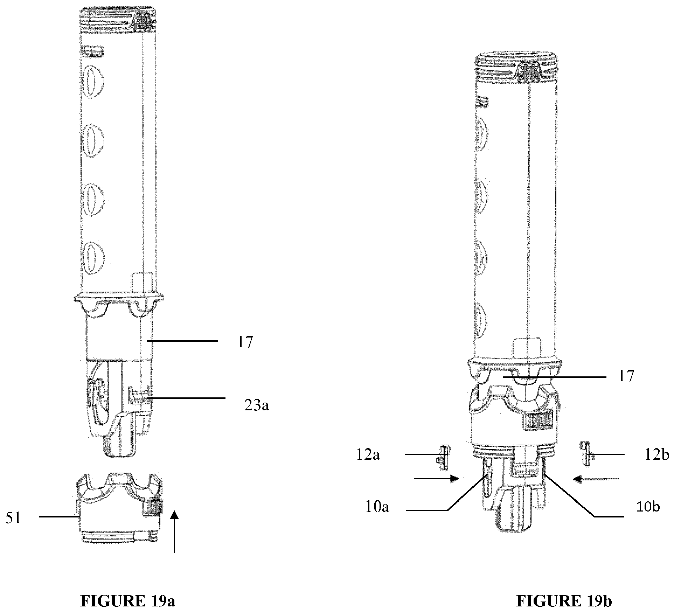

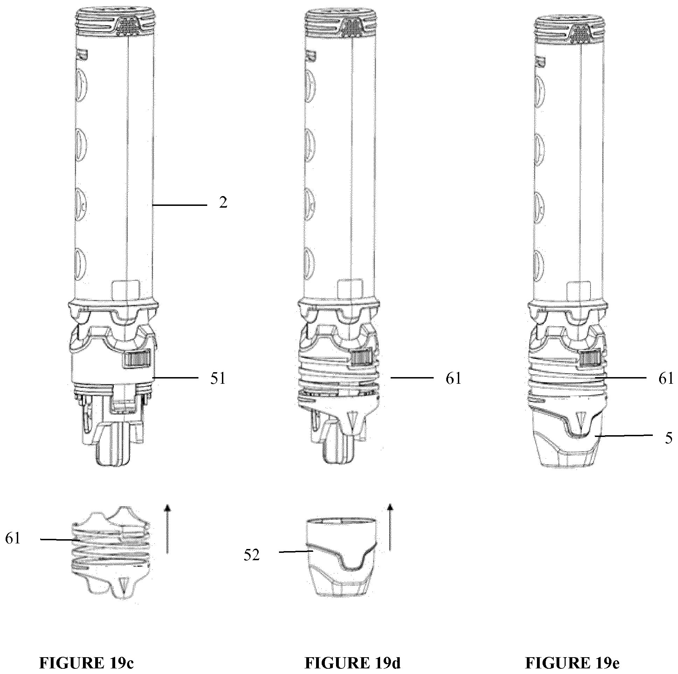

The locking projections **23a**, **23b** (as shown in **FIGURE 2**, **FIGURE 8** of aforementioned embodiment and **FIGURE 19a**) formed on the outer surface of second sleeve section **17** by groove cut diametrically opposite to the cam surfaces **22a**, **22b**. The locking projections **23a**, **23b** include a bevelled end surface extending radially outward which is shaped to lock with the reset members **59a**, **59b** (as shown in **FIGURE 17**) of cover member **5** when the cover member 5 is in the extended position.

The cam follower button **28** of the cam follower nuts **12a** and **12b** is engaged with protrusion head **25a** of cam track **24a** in the first locking position **13**. The cam follower button **28** of the cam follower nuts **12a** and **12b** abut with vertex **11d** on the cam track **24b** in the second locking position **14**.

The flex stem 7 (as shown in **FIGURE 1** and **FIGURE 7** of aforementioned embodiment) comprising a body defining a longitudinal central axis, four longitudinal blades **7a**, **7b**, **7c**, **7d** extending radially outward from the body, and opposed proximal and distal end portions. Further, the flex stem 7 has a base part **32** having a front face **32a** and a rear face **32b**, whereas the four longitudinal blades **7a**, **7b**, **7c**, **7d** affixed to the front face **32a** of the base part **32**.

A flex arm **8** extends from the rear face **32b** of the flex stem base part **32** towards the proximal end. A plurality of ribs **47a** also extend from the rear face **32b** of the flex stem base part **32**. Furthermore, a rib **47a'** extends from the periphery of rear face **32b**.

The flex arm **8** having a blade body having opposed first and second surfaces **8a**, **8b** that extends along a longitudinal axis of the blade body with the tapered end on the first surface **8a** of the blade body.

Prior to the use of trainer device **1**, the pin **50** of the safety cap **4** abuts with the tapered end of the flex arm **8** and obstructs the traversing of the flex arm **8** on the track **31** of the collet holder **3**.

The flex stem **7** has abutment with the cover member **5** and is held against spring 6 between the collet holder **3** and the cover member **5**, which restricts the movement of the cover member **5** prior to the use of trainer device 1.

The collet holder **3** (as shown in **FIGURE 3** and **FIGURE 9** of aforementioned embodiment) is a single piece body defining longitudinal central axis having, opposed upper and bottom surfaces **33** and **34**, opposed open distal end **35** and closed proximal end **36**, a pair of laterally opposed sides **37a**, **37b**, and an upper core member **38** with a top planar surface **38a** perpendicular to longitudinal central axis.

The collet holder **3** further comprises a pair of opposed projecting clips **39a**, **39b** extending downwardly from the upper core member **38** with radially outward protrusion heads **40a** and **40b** at the distal end. The collet holder **3** may be cylindrical, oval or elliptical in shape.

The bottom surface **34** of collet holder **3** having four corners **34a**, **34b**, **34c** and **34e**, a stop wall **41** extending upwardly from the two corners **34c**, **34d** of bottom surface **34** and being deflected outwardly at a region somewhat below the top-platen of collet holder **3** and disposed in common horizontal plane of upper core member **38**. The upper core member **38** having an opening **42** extends in a common horizontal plane at the central axis.

Further, the upper core member **38** of collet holder **3** has an opening **42** to receive the safety cap **4**.

The upper surface **33** of the collet holder **3** having a recess **43** extending longitudinally from open distal end **35** to the closed proximal end **36** comprising a stop wall **41**, and the stop wall **41** having front contact surface **41a**. The recess **43** with the stop wall **41** defining the track **31** for the flex arm **8**. Furthermore, the recess **43** formed in the upper surface **33** of the collet holder **3** having, a pair of opposed side surfaces **45a**, **45b** and central surface **46** extending parallel to the bottom surface **34** of collet holder **3** and ends in the stop wall **41**. The flex arm **8** at the distal end is in always contact and rests in the front contact surface **41a** of stop wall **41**.

The trainer device **1** of an autoinjector wherein the track **31** may be formed by a recess **43** extends longitudinally from open distal end **35** to the closed proximal end **36**.

At the distal end **35** of the collet holder **3**, the laterally opposite sides **37a**, **37b** have two inclined surfaces **44a**, **44b** perpendicular to each other.

A pair of diametrically opposed extended ribs **47b** extended from the open distal end **35** of the collet holder **3**, wherein one of the extended rib **47b** having curved cross-sectional recess at the middle of the rib body.

The rib **47a'** of the flex stem **7** and diametrically opposed extended ribs **47b** of the collet holder **3** define the spring platform **15** (as shown in **FIGURE** 7 and **FIGURE 9** of aforementioned embodiment), wherein the rib **47a'** fits in the curved cross-sectional recess of one of the extended rib **47b**. The spring platform **15** provides constant holding of the spring **6** during the operation of trainer device **1**.

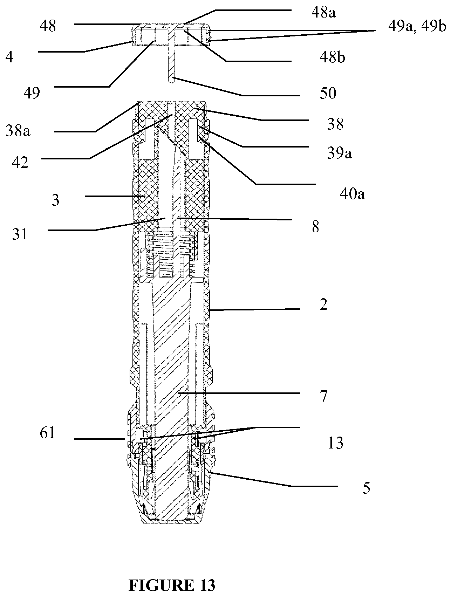

A safety cap **4** is removably connected to the collet holder **3** (as shown in **FIGURE 13**), the safety cap **4** comprises a flat top wall **48** having top planar surface **48a**, a bottom surface **48b**, a side wall **49** extending downwardly from the periphery of flat top wall **48** to the open free end and the inner surface **49a** and outer surface **49b**. The inner surface **49a** side wall **49** is sized to rest on the end surface **18** of housing **2**. Furthermore, the safety cap **4** comprising a pin **50** extending downwardly from the bottom surface **48b** of top wall **48** and removably engaged within an opening **42** in the upper core member **38** of the collet holder **3.**

To actuate the trainer device **1**, the user has to remove the safety cap **4** which allows the traversing of the flex arm **8** on the track **31** of the collet holder **3** during the operation.

As shown in **FIGURE 17**, the cover member **5** has two parts, an upper part **51** and a lower part **52**, whereas the upper part **51** and lower part **52** of cover member **5** are connected via plurality of rib and groove arrangements.

The upper part **51** of cover member **5** comprising, opposed proximal and distal end portions, whereas both the ends having the opening. A side wall **53** extending from a proximal end to a distal end around the periphery of the upper part, a plurality of ribs **54** separated by continuous grooves **55** extending downwardly along an inner circumference of the side wall **53** at the distal end in symmetrical arrangement. Further, a pair of diametrically opposed U-shaped like cross-sectional grooves **56** are provided on the side wall **53** at the proximal end.

The annular side wall **53** provides an outer bearing surface and having stopping ridge **53a** around the periphery of the upper part **51** of cover member **5** at the proximal end.

The lower part **52** of the cover member **5** comprising an opposed open proximal end and closed distal end. A bottom wall **56a** has a periphery defining the closed distal end and a side wall **56b** first extending in an annular fashion from the open proximal end followed by tapering frustoconical fashion and nests to periphery of bottom wall **56a.** The side wall **56b** provides an outer bearing surface and having stopping ridge **53b**.

A plurality of ribs **57** separated by continuous grooves **58** extending upwardly along the inner circumference of side wall **56b** at the proximal end to receive the corresponding ribs **54** and grooves **55** in the proper orientation of upper part **51** and lower part **52** of the cover member **5**. Diametrically opposed ledges **30a**, **30b** provided on the inner circumference of side wall **56b** below the grooves **58**. The ledges **30a**, **30b** having two edges. During the operation of trainer device **1**, the protrusion **27** of the cam follower nuts **12a** and **12b** moves rotationally from one edge to another edge of ledges **30a** and **30b**.

The inside diameter of annular side wall **56a** at the proximal end of lower part **52** of the cover member **5** is approximately equal to the outer diameter annular side wall **53** at the distal end of upper part **51** of cover member **5**.

In one preferred embodiment, the sleeve around the outer surface of the cover member **5** is a plastic sleeve **61** (as shown in **FIGURES 12-16**).

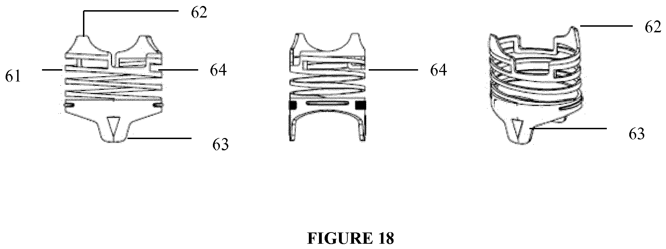

The plastic sleeve **61** will now be described in greater detail in connection with FIGURE **18**. The sleeve **61** may be a friction reducing sleeve provided over the cover member **5**. The plastic sleeve **61** may comprise plastic materials but not limited to thermosetting epoxy, polyimide, polyether ketone, polyester resins combinations thereof, and/or the like. The plastic sleeve **61** may include both a first end **62** and a second end **63**, wherein the first and second ends (**62**, **63**) are separated by coil body **64** extending along a coiled axis. The coil body **64** comprising a fibre- reinforced polymer material being substantially wound in one direction only helically and concentrically at prescribed winding angle.

The first end **62** of plastic sleeve **61** may be positioned around the bearing surface of the annular side wall **53** of upper part **51** of the cover member **5** and the stopping ridge **53a** prevents or inhibits the plastic sleeve **61** from extending beyond the stopping ridge **53a.** The second end **63** of plastic sleeve **61** may be positioned around the bearing surface of side wall **56b** of lower part **52** of the cover member 5 and the stopping ridge **53b** prevents or inhibits the plastic sleeve **61** from extending beyond the stopping ridge **53b**.

The plastic sleeve **61** may be at least partially coextensive with the cover member **5**, wherein the plastic sleeve **61** has friction-reducing internal surface relative to the outer bearing surface of both the upper part **51**, lower part **52** of the cover member **5** and is freely slidable between stopping ridges **53a** and **53b.**

As described herein, the trainer device **1** should be grasped proximal to the cover member **5**. The plastic sleeve **61** has an external surface against which the user's hand can be positioned as the user accidentally grasps the upper part **51** of the cover member **5** during actuation of the trainer device **1**. The plastic sleeve **61** may be in stationary position relative to user grasping, since the internal surface of the plastic sleeve **61** has low coefficient of friction that has slidable fit with the cover member **5** which allows the backward movement of the cover member **5** toward proximal end without any obstruction.

The coil body **64** acts as a helical spring and allows the compression of sleeve **61** when the proximal end **62** and the distal end **63** of the sleeve **61** are urged toward each other when the cover member **5** slidably moves towards proximal end. As the cover member **5** moves backward, the sleeve **61** gets compressed by the coil body **64** and maintains the sleeve **61** in constant contact with the cover member **5**.

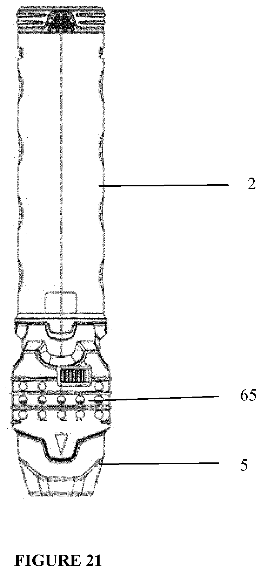

In another preferred embodiment, the sleeve around the outer surface of the cover member 5 is an elastomeric sleeve (as shown in FIGURE 21).

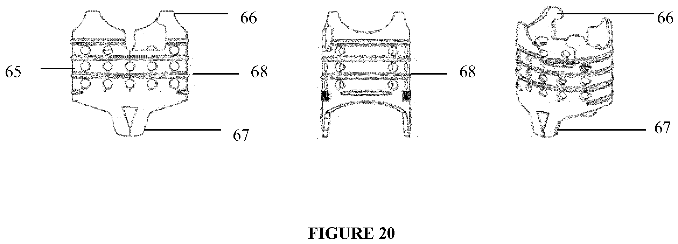

Referring to **FIGURE 20**, the elastomeric sleeve **65** may be an elongated friction reducing sleeve. The elastomeric sleeve **65** may be a friction reducing sleeve provided over the cover member **5**. The internal diameter and an internal surface of the elastomeric sleeve **65** that together provide a close but freely slidable fit with the cover member **5**. Referring to **FIGURE 20**, the elastomeric sleeve **65** may include both a first end **66** and a second end **67**, wherein the first and second ends (**66**, **67**) are separated by a tubular body **68** and the first and second ends may include openings.

The first end **66** of elastomeric sleeve **65** may be positioned around the bearing surface of the annular side wall **53** of upper part **51** of cover member **5** and the stopping ridge **53a** prevents or inhibits the elastomeric sleeve **65** from extending beyond the stooping ridge **53a**. The second end **67** of elastomeric sleeve **65** may be positioned around the bearing surface of side wall **56b** of lower part of cover member **5** and the stopping ridge **53b** prevents or inhibits the elastomeric sleeve from extending beyond the stopping ridge **53b**.

The elastomeric sleeve **65** may be at least partially coextensive with the cover member **5**, wherein the elastomeric sleeve **65** has friction-reducing internal surface relative to the outer bearing surface of both the upper part **51**, lower part **52** of cover member **5** and is freely slidable between stopping ridges **53a** and **53b**.

The elastomeric sleeve **65** may be formed of any suitable material that maintains low coefficient of static friction on both internal and external surfaces and resilient function. The desired coefficients of friction are below 0.5, and are preferably below 0.1.

The elastomeric sleeve **65** has an external surface against which the user's hand can be positioned as the user accidentally grasps the upper part **51** of cover member **5** during actuation of the trainer device **1**. The elastomeric sleeve **65** is in stationary position relative to user grasping, since the internal surface of the elastomeric sleeve **65** has slidable fit with the cover member **5** which allows the backward movement of the cover member **5** toward proximal end without any obstruction.

The tubular body **68** may be in elastomeric nature which acts as a spring and causes the compression of the sleeve **65** when the first end **66** and second end **67** of elastomeric sleeve **65** are urged towards each other when the cover member **5** slidably move towards proximal end of trainer device **1**. As the cover member **5** moves backward, the sleeve **65** gets compressed by the tubular elastomeric body **68** and maintains the sleeve **65** in constant contact with the cover member **5**.

After removing the cover member **5** from the body, the cover member **5** is extended forward towards distal end by the action of spring **6.** As the cover member **5** extended forwards, the elastomeric sleeve **65** is slipping from the user's hand and traverse with the cover member **5**.

During forward movement of the cover member **5**, the elastomeric sleeve **65** remains stationary relative to the outer bearing surface of cover member **5**.

The elastomeric sleeve **65** may comprise suitable material such as thermoset elastomers, for example natural rubbers, synthetic rubbers, thermoplastic elastomers and durable synthetic plastic material. As examples of synthetic rubbers, styrene- butadiene rubber, nitrile rubber, neoprene and ethylenepropylene rubbers, including copolymers and terpolymers such as ethylenepropylene terpolymer, can be mentioned. Examples of thermoplastic elastomers are styrene-butadiene-styrene, styrene-isoprene-styrene and styrene-ethylenebutylene block copolymers, as well as nylon types and olefinic, polyolefinic, polyester and polyurethane types.

The synthetic rubber also includes synthetic polyisoprene, polybutadiene, butadiene-styrene copolymers, butadiene-acrylonitrile copolymers, butadiene styrene acrylonitrile copolymers, polychloroprene and similar rubber products of 1, 3-diene monomers having a markedly reduced coefficient of friction.

The surface of the elastomeric or rubber article is directly treated and the surface structure thereof is modified whereby the coefficient of friction of the rubber surface is greatly reduced without alternating the essential elastic properties of the rubber article.

Suitable low friction coatings include self-lubricating materials, such as synthetic fluoropolymer, Nylon, or any other suitable low friction material or coating.

Examples of synthetic plastics materials are polyvinyl chloride and polyethylene. A plasticiser can be added to a plastic material makes sleeve flexible, and resilient in function. Examples of plasticisers are low molecular weight phthalates, high molecular weight phthalates and speciality plasticisers such as adipates, citrates, benzoates, trimelilates and polyoxyethylene additives such as acetal copolymers, polyacetal and polyformaldehyde.

The plastic sleeve **61** or an elastomeric sleeve **65** has a substantially circular or oval cross section.

The assembling procedures of the cover member **5** and the plastic sleeve **61** to the housing **2** of the trainer device 1 will now be described in greater detail in connection with **FIGURES 19a-19e****.** The upper part **51** of the cover member **5** is first positioned over the second sleeve section of housing **17** and securely placing the cam follower nuts **12a** and **12b** on the corresponding cam profiles **10a** and **10b** such that the cam follower buttons **28** are engaged with the first snap lock member **24**. The first end of the plastic sleeve **61** or the elastomeric sleeve **65** is affixed into the bearing surface of the upper part **51** of the cover member **5** and the stopping ridge **53a** provides that the plastic sleeve **61** or the elastomeric sleeve **65** does not extend beyond the outer periphery of the upper part **51** of the cover member **5**. After fixing the sleeve **61** or **65**, the lower part **52** is positioned into the upper part **51** of the cover member 5 and gets connected via plurality of rib and groove arrangements, such that the second end of the sleeve **61** or **65** is received over the outer surface of lower part **52** of the cover member **5**. The cam follower protrusions **27** are received in the ledges **30a, 30b** of the lower part **52** of the cover member **5**.

The assembling procedures of the cover member **5** and the elastomeric sleeve **65** to the housing **2** of the trainer device **1** will be in similar manner as described in **FIGURES 19a-19e****.**

As depicted in **FIGURE 11** of aforementioned embodiment, the cam follower nut **12a** moves on the cam profile **10a** during the various stages of the trainer device **1** for example the first locking position **13**, the actuation stage, the second locking position **14** and the resetting stage. In the first locking position **13**, the cam follower button **28** rests in the vertex **11a** of cam track **24a**. When the cover member **5** is moved backward, the cam follower button **28** traverses in the cam track **24a** from vertex **11a** to **11c** via **11b**; when the cover member **5** extends forward, the cam follower button **28** traverses in the cam track **24b** from vertex **11c** to vertex **11d** and the cover member **5** is in the second locking position **14**. Similarly, the cam follower nut **12b** follows the same movement on the diametrically opposed cam profile **10b**.

As described herein, for using the trainer device **1**, the safety cap **4** has to be removed first and the cover member **5** needs to be placed on the suitable part of the body, for example the upper leg, and is pressed. The lower part **52** and upper part **51** of the cover member **5** moves backwards towards the proximal end which results in a relative backward movement of the flex stem **7**. While the flex stem **7** is moved backwards, the flex arm **8** slidably traverses the front contact surface **41a** of stop wall **41** from side surface **45a** to **45b** of recess **43**. After removing the cover member **5** from the body, the cover member **5** extends forward towards the distal end by the action of spring **6**.

After using the trainer device **1**, it may be ready for reuse with a resetting mechanism **60**. The resetting mechanism **60** comprising: locking projections **23a, 23b** provided on the housing **2** (as shown in **FIGURE 19a**) and reset members **59a, 59b** provided on the outer sleeve **73** of top cover member **70** (as shown in **FIGURE 17**).

As shown in **FIGURE 17**, the reset members **59a** and **59b** is an elongated arm having upper and lower surfaces formed by groove cut on the surface of upper part **51** of the cover member **5**, the end portion of the reset members **59a, 59b** are locked with locking projections **23a, 23b** by abutting of lower surface of reset members **59a, 59b** with bevelled end surface of locking projection.

To reset the device, the user has to first place the safety cap **4** in the end surface **18** of housing **2** and presses the reset members **59a**, **59b** on the cover member **5**. Once the reset members **59a**, **59b** are pressed, the locking connection between the reset members **59a**, **59b** and the locking projections **23a, 23b** is released and the bevelled end portion of locking projection easily passes the reset member; then user pushes the cover member **5** back towards the proximal end from the second locking position **14** to first locking position **13** and the trainer device **1** ready for reuse. The present disclosure provides the resetting mechanism **60** which is separate from the locking mechanism **9** of the cover member **5** and does not disturb the cover member **5** in both retracted and extended positions.

In the trainer device **1** resetting operation, the cover member 5 moves backwards to first locking position **13,** as shown in **FIGURE 11** of aforementioned embodiment, the cam follower button **28** is traversing in cam track **24c** from vertex **11d** to **11a** via **11e** by passing of protrusion head **25a** of first snap lock member **25.**

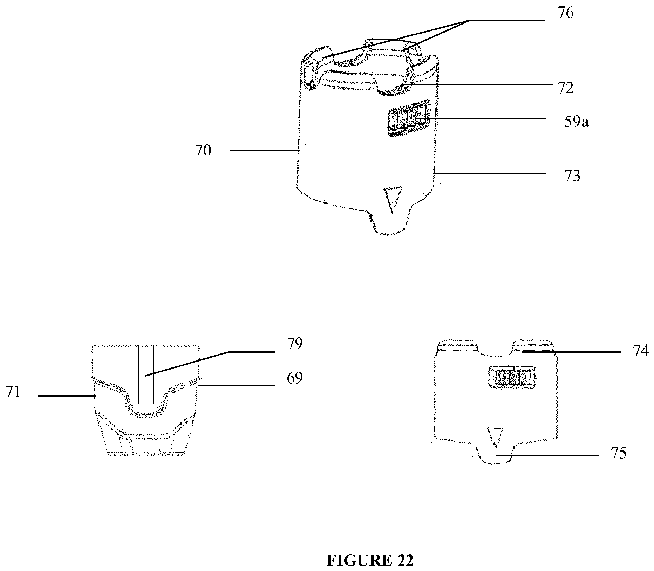

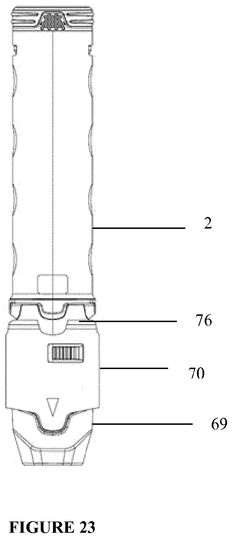

**FIGURE 22** and **FIGURE 23** depict an alternative embodiment of the cover member **5**. The cover member **5** has a base cover member **69** and a top cover member **70**. The base cover member **69** having opposed proximal and distal end portions, whereas both the ends having the opening and a side wall **71** extending from the proximal end to distal end. The top cover member **70** comprising an inner sleeve **72** and an outer sleeve **73**, arranged coaxially and concentrically. The top cover member **70** may include both a first **74** and a second end **75**. The concentrically arranged inner sleeve **72** and outer sleeve **73** are jointed together with an extending elastomeric part **76** at the first end **74**. The inner sleeve **72** of top cover member **70** comprising plurality of ribs **77a** separated by continuous grooves **77b** extending downwardly along an inner circumference at the distal end in symmetrical arrangement.

A plurality of ribs and continuous grooves **78a**, **78b** (not shown) extending upwardly along the inner circumference of base cover member **69** at the proximal end to receive the corresponding ribs and grooves **77a, 77b** (not shown) in the proper orientation of the base cover member **69** and the top cover member **70**.

The inside diameter of annular side wall **71** of base cover member **69** is substantially equal to the outer diameter of inner sleeve **72** of top cover member **70** in order to facilitate snap fit.

The cover member **5** further comprising a pair of diametrically opposed guiding channels **79** running on the outer surface of inner sleeve **72** of the top cover member **70** and continuous on the outer surface of the base cover member **69**. The guiding channels **79** extending from below the U-shaped like cross-sectional grooves **78** on the inner sleeve **72** of the top cover member **70** at the first end **74** to second end **75**, since the inside diameter of annular side wall **71** of base cover member **69** is substantially equal to the outer diameter of inner sleeve **72** of top cover member **70** allowing snap fit between them, the guiding channels **79** continues on the outer surface of the base cover member **69**.

The outer sleeve **73** of the top cover member **70** has a pair of diametrically opposed ribs **80** (not shown) on the inner surface corresponding to the guiding channels **79** on the outer surface of inner sleeve **72** of top cover member **70** and base cover member **69**. The ribs **80** and the guiding channels **79** arrangements facilitate the movement of outer sleeve **73** of top cover member **70** during the actuation of trainer device **1**.

The outer sleeve **73** of the top cover member **70** has an external surface against which the user's hand can be positioned as the user accidentally grasps the cover member **5** during actuation of the trainer device **1**. The outer sleeve **73** of the top cover member **70** is in stationary position relative to user grasping, since the outer sleeve **73** has joint with the inner sleeve **72** by the elastomeric part **76**, the ribs **80** and guiding channels **79** permit the movement of the inner sleeve **72** backwardly towards the proximal end by stretching of the elastomeric part **76** along with the base cover member **69**.

After removing the cover member **5** from the body, the cover member **5** is extended forward towards the distal end by the action of the spring **6**. As the inner sleeve **72** of the top cover member **70** along with the base cover member **69** extend forwards, the outer sleeve **73** slips from the user's hand and traverses with both the inner sleeve **72** and the base cover member **69**.

Various modifications and variations to the above-described trainer device 1 can be made without departing from the scope of the present claim 1.

## Claims

1. A trainer device **1** for an autoinjector comprising: (i) a housing **2**; (ii) a collet holder **3** that includes a track **31**; (iii) a safety cap **4**, comprising a pin **50**, which is removably connected to the collet holder 3; (iv) a cover member **5**; (v) a flex stem 7 having flex arm **8**; and (vi) a cover member locking mechanism **9** having a first locking position **13** and a second locking position **14**, wherein the housing **2** is partially received inside the cover member **5**, **characterized in that** the cover member locking mechanism **9** comprises cam profiles **10a,10b** and cam follower nuts **12a,12b,** wherein each of the cam profiles **10a,10b** has five vertices **11a,11b,11c,11d,11e** and comprises: (a) a first angled cam track **24a** formed by traversing of the cam profile **10a,10b** from vertex **11a** to vertices **11b,11c** towards the proximal direction; (b) a vertical cam track **24b** formed by traversing of the cam profile **10a,10b** from vertex **11c** to vertex **11d** in the distal direction; (c) a second angled cam track **24c** formed by traversing the cam profile **10a,10b** from the vertices **11d,11e** to vertex **11a** in the distal direction; (d) a first integral snap lock member **25** formed by groove cut on the surface of first angled cam track **24a** and comprising a protrusion head **25a** towards distal end extending radially outward having the sloping surface **25b** on the front projection surface; and (e) a second integral snap lock member **26** formed by groove cut on the surface of vertical cam track **24b** and comprising a protrusion head **26a** extending radially sideward having sloping surface **26b** on the front projection surface, wherein each of the cam follower nuts **12a,12b** comprises: (A) a cam follower protrusion **27** on the distal end portion; (B) a cam follower button **28** on the proximal end portion extending opposite to the cam follower protrusion **27**; (C) a cam follower body **29** extending between protrusion **27** and button **28**, wherein the cam follower button **28** of the cam follower nuts **12a,12b** is engaged with vertex **11a** on the first angled cam track **24a** in the first locking position **13** and the cam follower button **28** of the cam follower nuts **12a,12b** is engaged with vertex **11d** on the vertical cam track **24b** in the second locking position **14**, whereas integral snap lock members **25,26** provides the one-way traversing of cam follower nuts **12a,12b** in the cam profiles **10a,10b** during operation of trainer device **1**, while maintaining the cover member 5 in the first locking potion **13** and second locking position **14**.

2. The trainer device of claim 1, wherein the collet holder **3** comprises (i) a single piece body defining longitudinal central axis having opposed upper and bottom surfaces **33** and **34,** (ii) opposed open distal end **35** and a closed proximal end **36**, (iii) a pair of laterally opposed sides **37a, 37b,** and (iv) an upper core member **38** with a top planar surface **38a** perpendicular to longitudinal central axis, wherein the upper surface **33** of the collet holder **3** has a stop wall **41** and a recess **43** which extends longitudinally from open distal end **35** to the stop wall **41** and defining the track **31** for the flex arm **8**.

3. The trainer device of claim 1 comprising a resetting mechanism **60** for reuse of trainer device comprising: locking projections **23a,23b** provided on the housing **2** and reset members **59a,59b** provided on the cover member **5**, wherein locking connection between reset members **59a,59b** and locking projections **23a,23b** gets released when the reset members **59a,59b** are pressed by user which causes the bevelled end portion of locking projection to pass the reset member allowing the user to push back the cover member **5** towards the proximal end from the second locking position **14** to first locking position **13**.

4. The trainer device of claim 1, wherein the flex stem 7 has abutment with the cover member **5** and is held against the spring **6** between the collet holder **3** and the cover member **5**.

5. The trainer device of claim 1, wherein the pin **50** of the safety cap **4** abuts with the tapered end of flex arm **8** and obstructs the travelling of flex arm **8** on the track **31** of the collet holder **3**.

## Patentansprüche

1. Eine Trainingsvorrichtung 1 für einen Autoinjektor, umfassend: (i) ein Gehäuse 2; (ii) einen Klemmhalter 3, der eine Führung 31 aufweist; (iii) eine Sicherheitskappe 4, die einen Pin 50 aufweist, der lösbar mit dem Klemmhalter 3 verbunden ist; (iv) ein Abdeckelement 5; (v) einen flexiblen Schaft 7 mit einem flexiblen Arm 8; und (vi) einen Abdeckelement-Verriegelungsmechanismus 9 mit einer ersten Verriegelungsposition 13 und einer zweiten Verriegelungsposition 14, wobei das Gehäuse 2 teilweise innerhalb des Abdeckelements 5 angeordnet ist, **dadurch gekennzeichnet, dass** der Abdeckelement-Verriegelungsmechanismus 9 Nockenprofile 10a, 10b und Nockenmitnehmermuttern 12a, 12b umfasst, wobei jedes der Nockenprofile 10a, 10b fünf Spitzen 11a, 11b, 11c, 11d, 11e aufweist und umfasst: (a) eine erste abgewinkelte Nockenführung 24a, die durch Querbewegung des Nockenprofils 10a, 10b von der Spitze 11a zu den Spitzen 11b, 11c in proximaler Richtung gebildet wird; (b) eine vertikale Nockenführung 24b, die durch Querbewegung des Nockenprofils 10a, 10b von der Spitze 11c zu der Spitze 11d in distaler Richtung gebildet wird; (c) eine zweite abgewinkelte Nockenführung 24c, die durch Querbewegung des Nockenprofils 10a, 10b von den Spitzen 11d, 11e zu der Spitze 11a in distaler Richtung gebildet wird; (d) ein erstes integrales Schnappverriegelungselement 25, das durch einen Nutenschnitt auf der Oberfläche der ersten abgewinkelten Nockenführung 24a gebildet ist und einen sich zum distalen Ende hin radial nach außen erstreckenden Kopfvorsprung 25a mit einer schrägen Oberfläche 25b auf einer vorderen Projektionsfläche aufweist; und (e) ein zweites integrales Schnappverriegelungselement 26, das durch einen Nutenschnitt auf der Oberfläche der vertikalen Nockenführung 24b gebildet ist und einen sich radial seitwärts erstreckenden Kopfvorsprung 26a mit einer schrägen Oberfläche 26b auf einer vorderen Projektionsfläche aufweist, wobei jede der Nockenmitnehmermuttern 12a, 12b aufweist: (A) einen Nockenmitnehmervorsprung 27 am distalen Endabschnitt; (B) einen Nockenmitnehmerknopf 28 am proximalen Endabschnitt, der sich gegenüber dem Nockenmitnehmervorsprung 27 erstreckt; (C) einen Nockenmitnehmerkörper 29, der sich zwischen dem Vorsprung 27 und dem Knopf 28 erstreckt, wobei der Nockenmitnehmerknopf 28 der Nockenmitnehmermuttern 12a, 12b mit der Spitze 11a auf der ersten abgewinkelten Nockenführung 24a in der ersten Verriegelungsposition 13 in Eingriff ist und der Nockenmitnehmerknopf 28 der Nockenmitnehmermuttern 12a, 12b mit der Spitze 11d auf der vertikalen Nockenführung 24b in der zweiten Verriegelungsposition 14 in Eingriff steht, wobei die integrierten Schnappverriegelungselemente 25, 26 die einseitige Verschiebung der Nockenmitnehmermuttern 12a, 12b in den Nockenprofilen 10a, 10b während des Betriebs der Trainingsvorrichtung 1 ermöglichen, während sie das Abdeckelement 5 in der ersten Verriegelungsposition 13 und der zweiten Verriegelungsposition 14 halten.

2. Trainingsvorrichtung nach Anspruch 1, wobei der Klemmhalter 3 umfasst: (i) einen einstückigen Körper, der eine zentrale Längsachse definiert und sich gegenüberliegende obere und untere Oberflächen 33 und 34 aufweist, (ii) ein sich gegenüberliegendes offenes distales Ende 35 und ein geschlossenes proximales Ende 36, (iii) ein Paar sich seitlich gegenüberliegende Seiten 37a, 37b, und (iv) ein oberes Kernelement 38 mit einer oberen ebenen Fläche 38a senkrecht zu der zentralen Längsachse, wobei die obere Oberfläche 33 des Klemmhalters 3 eine Anschlagswand 41 und eine Aussparung 43 aufweist, der sich in Längsrichtung von dem offenen distalen Ende 35 zu der Anschlagswand 41 erstreckt und die Führung 31 für den flexiblen Arm 8 bildet.

3. Trainingsvorrichtung nach Anspruch 1, umfassend einen Rückstellmechanismus 60 zur Wiederverwendung der Trainingsvorrichtung, umfassend: Verriegelungsvorsprünge 23a, 23b an dem Gehäuse 2 und Rückstellelemente 59a, 59b an dem Abdeckelement 5, wobei die Verriegelungsverbindung zwischen den Rückstellelementen 59a, 59b und den Verriegelungsvorsprüngen 23a, 23b gelöst wird, wenn die Rückstellelemente 59a, 59b von einem Benutzer betätigt werden, was bewirkt, dass der abgeschrägte Endabschnitt des Verriegelungsvorsprungs an dem Rückstellelement vorbeigeführt wird, was es dem Benutzer ermöglicht, das Abdeckelement 5 in Richtung des proximalen Endes aus der zweiten Verriegelungsposition 14 in die erste Verriegelungsposition 13 zurückzuschieben.

4. Trainingsvorrichtung nach Anspruch 1, wobei der flexible Schaft 7 an dem Abdeckelement 5 anliegt und gegen die Feder 6 zwischen dem Klemmhalter 3 und dem Abdeckelement 5 gedrückt wird.

5. Trainingsvorrichtung nach Anspruch 1, wobei der Pin 50 der Sicherheitskappe 4 an dem verjüngten Ende des flexiblen Arms 8 anliegt und die Bewegung des flexiblen Arms 8 auf der Führung 31 des Klemmhalters 3 sperrt.

## Revendications

1. Dispositif d'entraînement (1) pour un injecteur automatique comprenant : (i) un boîtier (2) ; (ii) un porte-douille (3) qui comprend une piste (31) ; (iii) un capuchon de sécurité (4), comprenant une broche (50), qui est reliée de manière amovible au porte-douille (3) ; (iv) un élément de recouvrement (5) ; (v) une tige flexible (7) ayant un bras flexible (8) ; et (vi) un mécanisme de verrouillage d'élément de recouvrement (9) ayant une première position de verrouillage (13) et une seconde position de verrouillage (14), le boîtier (2) étant partiellement reçu à l'intérieur de l'élément de recouvrement (5), **caractérisé par le fait que** le mécanisme de verrouillage d'élément de recouvrement (9) comprend des profils de came (10a, 10b) et des écrous de galet suiveur (12a, 12b), chacun des profils de came (10a, 10b) ayant cinq sommets (11a, 11b, 11c, 11d, 11e) et comprenant : (a) une première piste de came angulaire (24a) formée par traversée du profil de came (10a, 10b) du sommet (11a) aux sommets (11b, 11c) vers la direction proximale ; (b) une piste de came verticale (24b) formée par traversée du profil de came (10a, 10b) du sommet (11c) au sommet (11d) dans la direction distale ; (c) une seconde piste de came angulaire (24c) formée par traversée du profil de came (10a, 10b) des sommets (11d, 11e) au sommet (11a) dans la direction distale ; (d) un premier élément de verrouillage à encliquetage intégral (25) formé par une rainure découpée sur la surface de la première piste de came angulaire (24a) et comprenant une tête en saillie (25a) vers une extrémité distale s'étendant radialement vers l'extérieur ayant la surface inclinée (25b) sur la surface de saillie avant ; et (e) un second élément de verrouillage à encliquetage intégral (26) formé par une rainure découpée sur la surface de la piste de came verticale (24b) et comprenant une tête en saillie (26a) s'étendant radialement sur le côté ayant une surface inclinée (26b) sur la surface de saillie avant, chacun des écrous de galet suiveur (12a, 12b) comprenant : (A) une saillie de galet suiveur (27) sur la partie d'extrémité distale ; (B) un bouton de galet suiveur (28) sur la partie d'extrémité proximale s'étendant à l'opposé de la saillie de galet suiveur (27) ; (C) un corps de galet suiveur (29) s'étendant entre la saillie (27) et le bouton (28), le bouton de galet suiveur (28) des écrous de galet suiveur (12a, 12b) étant engagé avec le sommet (11a) sur la première piste de came angulaire (24a) dans la première position de verrouillage (13) et le bouton de galet suiveur (28) des écrous de galet suiveur (12a,12b) étant engagé avec le sommet (11d) sur la piste de came verticale (24b) dans la seconde position de verrouillage (14), tandis que les éléments de verrouillage à encliquetage intégral (25, 26) assurent la traversée à sens unique des écrous de galet suiveur (12a, 12b) dans les profils de came (10a, 10b) pendant le fonctionnement du dispositif d'entraînement (1), tout en maintenant l'élément de recouvrement (5) dans la première position de verrouillage (13) et la seconde position de verrouillage (14).

2. Dispositif d'entraînement selon la revendication 1, dans lequel le porte-douille (3) comprend (i) un corps d'une seule pièce définissant un axe central longitudinal ayant des surfaces supérieure et inférieure opposées (33 et 34), (ii) une extrémité distale ouverte (35) et une extrémité proximale fermée (36) opposées, (iii) une paire de côtés latéralement opposés (37a, 37b), et (iv) un élément central supérieur (38) avec une surface plane supérieure (38a) perpendiculaire à l'axe central longitudinal, dans lequel la surface supérieure (33) du porte-douille (3) a une paroi d'arrêt (41) et un évidement (43) qui s'étend longitudinalement de l'extrémité distale ouverte (35) à la paroi d'arrêt (41) et définissant la piste (31) pour le bras flexible (8).

3. Dispositif d'entraînement selon la revendication 1, comprenant un mécanisme de réinitialisation (60) pour la réutilisation du dispositif d'entraînement comprenant : des saillies de verrouillage (23a, 23b) disposées sur le boîtier (2) et des éléments de réinitialisation (59a, 59b) disposés sur l'élément de recouvrement (5), dans lequel la liaison de verrouillage entre les éléments de réinitialisation (59a, 59b) et les saillies de verrouillage (23a, 23b) est libérée lorsque les éléments de réinitialisation (59a, 59b) sont pressés par l'utilisateur, ce qui amène la partie d'extrémité biseautée de la saillie de verrouillage à passer l'élément de réinitialisation permettant à l'utilisateur de repousser l'élément de recouvrement (5) vers l'extrémité proximale de la seconde position de verrouillage (14) à la première position de verrouillage (13).

4. Dispositif d'entraînement selon la revendication 1, dans lequel la tige flexible (7) est en butée avec l'élément de recouvrement (5) et est maintenue contre le ressort (6) entre le porte-douille (3) et l'élément de recouvrement (5).

5. Dispositif d'entraînement selon la revendication 1, dans lequel la broche (50) du capuchon de sécurité (4) vient en butée avec l'extrémité effilée du bras flexible (8) et empêche le déplacement du bras flexible (8) sur la piste (31) du porte-douille (3).
